# EUROPEAN PATENT APPLICATION

(11) **EP 1 889 618 A1**
(43) Date of publication of application: **20.02.2008**
(21) Application number: 06746879.3
(22) Date of filing: 26.05.2006
(51) Int. Cl.: A61K 31/427, A61K 45/00, A61P 3/10, A61P 5/50, A61P 43/00

(54) **COMBINED DRUG FOR TREATING DIABETES**

(30) Priority: 27.05.2005 JP 2005155174
(71) Applicant: Daiichi Sankyo Company, Limited, Tokyo 103-8426 (JP)
(72) Inventor: NAKASHIMA, Ryutaro, 1-2-58, Hiromachi,Shinagawa-ku,Tokyo 140-8710 (JP); OGAWA, Junko, 1-2-58, Hiromachi,Shinagawa-ku,Tokyo 140-8710 (JP); OKUNO, Akira, 1-2-58, Hiromachi,Shinagawa-ku,Tokyo 140-8710 (JP)
(74) Representative: Gibson, Christian John Robert
(86) International application number: PCT/JP2006/310550
(87) International publication number: WO 2006/126673

(57) **Abstract**

The present invention provides a pharmaceutical composition (preferably a drug for therapeutic and/or prophylactic treatment of diabetes) comprising a GLP-1 receptor agonist and the insulin resistance improving agent 5-[4-(6-methoxy-1-methyl-1H-benzimidazol-2-ylmethoxy)benzyl]thiazolidine-2,4-dione, or a pharmacologically acceptable salt thereof, in combination.

## Description

### Technical Field

The present invention relates to a pharmaceutical composition (preferably a drug for therapeutic and/or prophylactic treatment of diabetes) comprising a GLP-1 receptor agonist and the insulin resistance improving agent 5-[4-(6-methoxy-1-methyl-1H-benzimidazol-2-ylmethoxy)benzyl]thiazolidine-2,4-dione, or a pharmacologically acceptable salt thereof, in combination.

Furthermore, the present invention relates to use of the above-mentioned compound for production of the above-mentioned pharmaceutical composition and to a method for prophylactic or therapeutic treatment of the above-mentioned disease, characterized in that the above-mentioned medicament is administered to a warm-blooded animal (preferably a human).

### Background Art

Diabetes is a chronic metabolic disorder which presents high blood sugar levels as the main indicator. It is said that there are about 6.9 million patients in Japan and about 150 million patients worldwide as of 1999, and the number of patients tends to increase further each year. Development of therapies for this disease is a critical issue.

Prolonged chronic high blood sugar levels due to diabetes lead to dysfunctions of pancreatic P cells, such as decreases of the cells, which cells synthesize and secrete insulin, which plays an important role in control of blood sugar levels, resulting in persistent and severe high blood sugar levels (for example, refer to Non-Patent Documents 1 and 2). Accordingly, in recent years, there has been a particularly strong desire for development of a therapeutic drug for diabetes that can control blood sugar levels over a long period while protecting pancreatic β cells, for the prevention of severe diabetes.

Glucagon-like peptide-1 (GLP-1) is a peptide having a sequence found at the C terminus of mammalian proglucagon, and various fragments thereof (for example, GLP-1 (7-37), GLP-1 (7-36) amide, derivatives and analogues thereof, etc.) are known to act on β cells in the pancreatic islets of Langerhans and thereby exert bioactivities such as an insulinotropic action and a pancreatic β cell protecting action (for example, refer to Patent Documents 1 and 2). A wide variety of derivatives and analogues including exendin 4 and NN2211 have so far been developed as GLP-1-related peptides, and nausea, vomiting, loss of appetite, and so forth are known as adverse drug reactions characteristic to these peptides.

Meanwhile, insulin resistance improving agents are known to lower blood sugar levels by improving an impaired insulin action and improve dysfunctions of pancreatic β cells such as reduced pancreatic insulin content, which is observed in the diabetic condition (for example, refer to Non-Patent Documents 3 and 4). As insulin resistance improving agents, rosiglitazone and pioglitazone, which are thiazolidinedione compounds, are currently marketed, and cardiac hypertrophy, pleural effusion retention, edema, and so forth due to body fluid retention are known as adverse drug reactions characteristic thereto.

Combined use of a thiazolidinedione compound as an insulin resistance improving agent and a GLP-1 receptor agonist in diabetes treatment has been reported in Patent Document 3 and Non-Patent Document 5.

Patent Document 3 mentions that a synergistic therapeutic effect against diabetes can be achieved by administering a thiazolidinedione compound and a GLP-1 receptor agonist in combination. However, since the actual biological data has only disclosed that combined use of TZD300512, a thiazolidinedione compound, and IP⁷, a GLP-1 (7-37) derivative, exhibited an additive effect on various parameters such as body weight, food intake, blood glucose levels, blood insulin levels, glycosylated hemoglobin (HbA1c) levels, and heart weight in diabetes model rats, but not a synergistic effect, it is not recognized from the disclosure in this document that combined use of a thiazolidinedione compound and a GLP-1 receptor agonist actually exhibits a synergistic effect in diabetes treatment.

Furthermore, Non-Patent Document 5 published after the publication of Patent Document 3 has reported that combined use of pioglitazone, a thiazolidinedione compound, and GLP-1 exhibited an additive therapeutic effect against diabetes as shown in parameters such as blood sugar levels immediately post-meal, blood sugar levels at 8 hours post-meal, blood insulin levels, and blood glucagon levels.

Accordingly, exhibition of an additive effect by a thiazolidinedione compound and a GLP-1 receptor agonist in the treatment of type-2 diabetes is the technological level in the research field of diabetes, and no combination of a thiazolidinedione compound and a GLP-1 receptor agonist is known that exhibits a synergistic effect in the treatment of diabetes.
Patent Document 1: WO90/11296
Patent Document 2: WO91/11457
Patent Document 3: WO00/78333
Non-Patent Document 1: Endocrine Reviews (U.S.), Vol. 13, pp. 415-431 (1992)
Non-Patent Document 2: Diabetes (U.S.), Vol. 43, pp. 1085-1089 (1994)
Non-Patent Document 3: Metabolism (U.S.), Vol. 53, No. 4, pp. 488-494 (2004)
Non-Patent Document 4: Diabetes (U.S.), Vol. 50, pp. 1021-1029 (2001)
Non-Patent Document 5: Diabetes Care (U.S.), Vol. 27, pp. 1910-1914 (2004)

### Disclosure of the Invention

The inventors of the present invention conducted various researches in order to develop a therapeutic method for diabetes that has an effect of improving dysfunctions of pancreatic P cells and enables long-term control of blood sugar levels. As a result, they found that this object could be achieved by using a GLP-1 receptor agonist and the insulin resistance improving agent 5-[4-(6-methoxy-1-methyl-1H-benzimidazol-2-ylmethoxy)benzyl]thiazolidine-2,4-dione, or a pharmacologically acceptable salt thereof, in combination, and accomplished the present invention.
The present invention relates to the following:
(1) A pharmaceutical composition comprising a GLP-1 receptor agonist and 5-[4-(6-methoxy-1-methyl-1H-benzimidazol-2-ylmethoxy)benzyl]thiazolidine-2,4-dione or a pharmacologically acceptable salt thereof in combination.
(2) The pharmaceutical composition according to (1), wherein the GLP-1 receptor agonist is a GLP-1 related peptide.
(3) The pharmaceutical composition according to (2), wherein the GLP-1 related peptide is GLP-1 (7-37), GLP-1 (7-36) amide, Arg³⁴Lys²⁶(N⁶-(γ-L-glutamyl(N^{α}-pahnitoyl))) GLP-1 (7-37), exendin 3, exendin 4, a derivative or analogue thereof, or a pharmaceutically acceptable salt thereof.
(4) The pharmaceutical composition according to (2), wherein the GLP-1 related peptide is GLP-1 (7-37), GLP-1 (7-36) amide, Arg³⁴Lys²⁶(N⁶-(γ-L-glutamyl(N^{α}-palmitoyl))) GLP-1 (7-37), exendin 3, exendin 4, or a pharmaceutically acceptable salt thereof.
(5) The pharmaceutical composition according to (2), wherein the GLP-1 related peptide is GLP-1 (7-36) amide or a pharmaceutically acceptable salt thereof.
(6) The pharmaceutical composition according to any one of (1) to (4), characterized in that the GLP-1 receptor agonist is administered via an administration route selected from intravenous, intranasal, and subcutaneous routes, and 5-[4-(6-methoxy-1-methyl-1H-benzimidazol-2-ylmethoxy)benzyl]thiazolidine-2,4-dione or a pharmacologically acceptable salt thereof is administered via the oral administration route.
(7) The pharmaceutical composition according to any one of (1) to (6), which is a drug for prophylactic or therapeutic treatment of diabetes.
(8) The pharmaceutical composition according to (7), which is a glycosylated hemoglobin lowering agent.
(9) The pharmaceutical composition according to (7), which is a blood sugar lowering agent.
(10) The pharmaceutical composition according to (7), which is an agent for improving pancreatic β cell function.
(11) The pharmaceutical composition according to (7), which is an insulin resistance improving agent.
(12) Use of a GLP-1 receptor agonist and 5-[4-(6-methoxy-1-methyl-1H-benzimidazol-2-ylmethoxy)benzyl]thiazolidine-2,4-dione or a pharmacologically acceptable salt thereof for production of the pharmaceutical composition according to any one of (1) to (11).
(13) Use of a GLP-1 receptor agonist or 5-[4-(6-methoxy-1-methyl-1H-benzimidazol-2-ylmethoxy)benzyl]thiazolidine-2,4-dione or a pharmacologically acceptable salt thereof for production of the pharmaceutical composition according to any one of (1) to (11).
(14) A method of therapeutic or prophylactic treatment of diabetes, characterized in that a GLP-1 receptor agonist and 5-[4-(6-methoxy-1-methyl-1H-benzimidazol-2-ylmethoxy)benzyl]thiazolidine-2,4-dione or a pharmacologically acceptable salt thereof are administered in combination to a patient with diabetes or a patient who is at risk of having diabetes.

In the present invention, the term "GLP-1 receptor agonist" means a substance that binds to the GLP-1 receptor expressed on pancreatic P cells to exert an insulinotropic action or a pancreatic P cell protecting action in the pancreas, and the GLP-1 receptor agonists are not particularly limited so long as they have such activity (hereinafter, referred to as "GLP-1 activity").

One example of a GLP-1 receptor agonist is a GLP-1 related peptide. In the present invention, the term "GLP-1 related peptide" means a peptide that has homology with the amino acid sequence of SEQ ID NO: 1 in the sequence listing, a fragment thereof, a derivative thereof, or a pharmacologically acceptable salt thereof, and that has GLP-1 activity. Specific examples of GLP-1 related peptides include GLP-1 (7-37), GLP-1 (7-36) amide, derivatives thereof, analogues thereof, exendin peptides, and so forth. Furthermore, pharmacologically acceptable salts of the above-mentioned peptides are also included in the GLP-1 related peptides of the present invention. GLP-1 (7-37) is a peptide having the amino acid sequence of SEQ ID NO: 1 in the sequence listing. GLP-1 (7-36) amide has an amino acid sequence comprising amino acid numbers 1 to 30 of SEQ ID NO: 1 in the sequence listing, and is a peptide obtained by amidating the carboxyl group of Arg at the C terminus. Both GLP-1 (7-37) and GLP-1 (7-36) amide can be produced according to the descriptions in U.S. Patent No. 5614,492 or WO91/114

In the present invention, the term "derivative" as used for a peptide means a substance with a modified amino acid or modified amino acids constituting the peptide. Modification as used herein may be chemical modification, modification by microbial transformation, or the like. Modified sites are not particularly limited, and a side chain of an amino acid, the α carbon of an amino acid, or the amino group or carboxyl group bound thereto may be modified. Preferably, however, a side chain of an amino acid, the amino group at the N terminus of the peptide, or the carboxyl group at the C terminus of the peptide is modified. Since GLP-1 (7-37) and GLP-1 (7-36) amide are rapidly biodegraded in the blood, studies on their derivatization for making them less susceptible to such degradation have been actively conducted. As a result, various derivatives of GLP-1 (7-37) and GLP-1 (7-36) amide are known. Such derivatives can be produced according to, for example, the descriptions in WO90/11296, WO91/11457, or the like. These derivatives are also included in the GLP-1 related peptides of the present invention so long as they have GLP-1 activity.

The term "analogue" as used for a peptide in the present invention refers to a peptide having the amino acid sequence of a natural peptide that includes substitution, deletion, insertion, or addition of one or more amino acids. The upper limit of the number of amino acids substituted, deleted, inserted, or added in the analogue of the present invention is not particularly limited so long as the GLP-1 activity is maintained, but is preferably 10, more preferably 5, yet more preferably 3, yet more preferably 2. The form of amino acid modification may be any of substitution, deletion, insertion, or addition, but is preferably substitution or deletion at a terminus, more preferably substitution. Since GLP-1 (7-37) and GLP-1 (7-36) amide are rapidly biodegraded in blood, studies on their conversion to analogues for making them less susceptible to such degradation have been actively conducted. As a result, various analogues of GLP-1 (7-37) and GLP-1 (7-36) amide are known. Such analogues can be produced according to, for example, the descriptions in WO90/11296, WO91/11457, or the like. These analogues are also included in the GLP-1 related peptides of the present invention so long as they have GLP-1 activity.

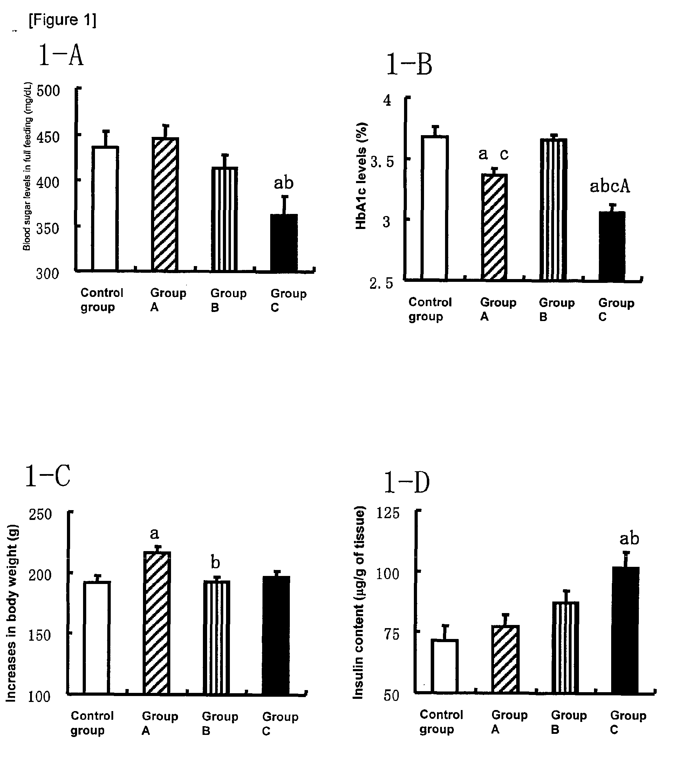

Derivatives of the analogues of GLP-1 (7-37) or GLP-1 (7-36) amide are also included in the GLP-1 related peptides of the present invention so long as they have GLP-1 activity. Such peptides are disclosed in WO90/11296, WO91/114 and the like and can be produced according to the descriptions in these publications. Such peptides are not particularly limited so long as they have GLP-1 activitiy, but preferably have 3 or less, more preferably 2 or less substituted amino acids. Examples of such peptides include Arg³⁴Lys²⁶N⁶-(γ-L-glutamyl(N^{α}-palmitoyl))) GLP-1 (7-37) (a compound having the structure shown in Figure 1 in the paper of Jesper et al. [Jesper et al., Journal of Labelled Compounds and Radiopharmaceuticals, 2003, Vol. 46, pp. 499-510]: nonproprietary name, liraglutide). Arg³⁴Lys²⁶(N⁶-(γ-L-glutamyl(N^{α}-palmitoyl))) GLP-1 (7-37) can be produced according to the descriptions in the above-mentioned paper of Jesper et al. or WO98/08871. Furthermore, a preparation containing this substance can be produced according to the description in W003/2136, for example.

In the present invention, exendin peptides are also included in the GLP-1 related peptides. Exendin peptides are a group of peptides extracted from lizard venom that have a homology with GLP-1 peptide. The exendin peptides are not particularly limited so long as they have GLP-1 activity, but are preferably exendin 3 (SEQ ID NO: 3) or exendin 4 (SEQ ID NO: 2; nonproprietary name, exenatide), more preferably exendin 4. Exendin 3 and exendin 4 can be produced according to the descriptions in Japanese Patent Application Publication (kouhyou) No. 2002-534450, U.S. Patent Application Publication No. 2003-87820, and European Patent No. 1140145.

In the field of peptides, it is generally said that an original peptide and a peptide differing from the original peptide even by one amino acid residue do not necessarily have identical activity. However, GLP-1 peptide has been well-studied in order to improve its property of disappearance from the blood, and a very wide variety of derivatives, analogues, similar peptides, and the like are known to have the GLP-1 activity (for example, refer to WO90/11296, WO91/11457, etc.). In the present invention, GLP-1 related peptides include a wide range of peptides. GLP-1 related peptides are not particularly limited so long as they have GLP-1 activity, but are preferably GLP-1 (7-37), GLP-1 (7-36) amide, Arg³⁴Lys²⁶(N⁶-(γ-L-glutamyl(N^{α}-palmitoyl))) GLP-1 (7-37), exendin 3, exendin 4, derivatives or analogues thereof, or pharmaceutically acceptable salts thereof, more preferably GLP-1 (7-37), GLP-1 (7-36) amide, Arg³⁴Lys²⁶(N⁶-(γ-L-glutamyl(N^{α}-palmitoyl))) GLP-1 (7-37), exendin 3, exendin 4, or pharmaceutically acceptable salts thereof, yet more preferably GLP-1 (7-36) amide.

In the present invention, one kind of GLP-1 receptor agonist is usually used, but two or more kinds of GLP-1 receptor agonists can be used in combination.

In the present invention, "insulin resistance improving agent" is a generic term for drugs that improve insulin resistance and enhance insulin sensitivity. 5-[4-(6-Methoxy-1-methyl-1 H-benzimidazol-2-ylmethoxy)benzyl]thiazolidine-2,4-dione (hereinafter, also referred to as "compound A"), an insulin resistance improving agent, and salts thereof can be produced according to the methods described in Japanese Patent Laid-Open No. 9-295970, European Patent No. 0745600, U.S. Patent No. 5,886,014, and International Patent Publication WO00/71540.

In the present invention, "a pharmaceutical composition comprising (two compounds) in combination" means a pharmaceutical composition intended to be used such that one compound should be administered during a period of disease treatment using the other one compound. Here, these two drugs may be administered simultaneously or separately with an interval. Furthermore, an administration route suitable for each drug is selected as the administration route. In this case, the method of administering two drugs separately may also be referred to as "combined use". Furthermore, separate administration of a GLP-1 receptor agonist and compound A or a pharmacologically acceptable salt thereof is referred to as "the combined use of the present invention."

In the present invention, the term "additive effect" means that, in a test, an effect of two or more drugs administered in combination (the difference between the value of a combination treatment group and that of a control group) does not exceed the sum of effects shown in the respective test results of single drugs (the difference between the value of a single drug treatment group and that of a control group).

In the present invention, the term "synergistic effect" means that, in a test, an effect of two or more drugs administered in combination (the difference between the value of a combination treatment group and that of a control group) exceeds the sum of effects shown in the respective test results of single drugs (the difference between the value of a single drug treatment group and that of a control group). This case is referred to as a synergistic effect even though no statistically significant difference is observed. Preferably, however, a synergistic effect is shown with a statistically significant difference. A synergistic effect with a statistically significant difference can be shown by two-way analysis of variance.

A GLP-1 receptor agonist and compound A can be administered in the form of a combination drug. Furthermore, these single drugs can be simultaneously administered. Furthermore, these single drugs can be administered at a suitable interval. Administration intervals acceptable to achieve the effect of such drug administration are not particularly limited and can be confirmed by clinical or nonclinical experiment. It is desirable to administer single drugs at intervals suitable for administration of each drug, and the administration interval of each single drug is determined in a nonclinical or clinical test of each single drug.

Each single drug is prepared and administered in a form suitable for each substance, and the administration routes may be different from each other. Thus, the drug administration according to the administration route, dosage form, and administration interval suitable for each drug is also included in the combined use of the present invention, and a pharmaceutical composition comprising a GLP-1 receptor agonist and/or compound A that is produced with the intention of this combined use is also included in the present invention.

The pharmaceutical composition of the present invention is administered via various routes. The administration routes are not particularly limited and determined depending on the dosage form, patient's age, sex, and other conditions, severity of the disease, and the like. For example, tablet, pill, powder, granule, syrup, solution, suspension, emulsion, granule, and capsule are orally administered. Furthermore, the GLP-1 receptor agonist and compound A may be contained in one formulation, or formulations each containing either of them separately may be used in combination.

These various formulations can be prepared according to usual methods by using known agents commonly used in the known field of pharmaceutical formulation, such as excipients, binders, disintegrants, lubricants, solubilizing agents, flavoring agents, and coating agents in addition to an active ingredient.

To prepare a tablet form, a wide range of known substances used in this field can be used as a carrier, and examples thereof include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrants such as dry starch, sodium alginate, powdered agar, laminaran powder, sodium hydrogencarbonate, calcium carbonate, polyoxyethylene sorbitan fatty acid esters, lauryl sodium sulfate, monoglyceride stearate, starch, and lactose; disintegration inhibitors such as sucrose, stearin, cocoa butter, and hydrogenated oils; absorption enhancers such as quaternary ammonium bases and lauryl sodium sulfate; moisturizing agents such as glycerine and starch; adsorbents such as starch, lactose, kaolin, bentonite, and colloidal silicic acid; lubricants such as purified talc, stearate, fluoboric acid powder, and polyethylene glycol, and so forth. Furthermore, tablets can be prepared as usual coated tablets as required, and examples thereof include sugar-coated tablet, gelatin-coated tablet, enteric-coated tablet, film-coated tablet, double-layered tablet, and multi-layered tablet.

To prepare a pill form, a wide range of known substances used in this field can be used as a carrier, and examples thereof include excipients such as glucose, lactose, starch, cacao butter, hydrogenated vegetable oil, kaolin, and talc; binders such as gum arabic powder, tragacanth powder, gelatin, and ethanol; disintegrants such as laminaran and agar, and so forth.

Furthermore, if necessary, coloring materials, preservatives, flavors, flavoring agents, sweeteners, other drugs, and the like may be added.

The amount of an active ingredient compound contained in the above-mentioned pharmaceutical formulations is not particularly limited, but is suitably selected in a wide range. It is usually suitable to include the active ingredient compound in an amount of 1 to 70% by weight, preferably 1 to 30% by weight, of the total composition.

The dose of each drug used in the present invention and the administration ratio vary greatly depending on various factors such as the activity of each substance and the patient's symptoms, age, and body weight.

The amount of GLP-1 receptor agonist contained in the above-mentioned pharmaceutical formulations is not particularly limited but is suitably selected in a wide range. It is usually suitable to contain the GLP-1 receptor agonist in an amount of 1 to 70% by weight, preferably 1 to 30% by weight, of the total composition.

The dose varies depending on symptoms, age, body weight, dosage form, and the like, and the usual daily dose for an adult is at least 0.001 mg or 0.00002 mg/kg (preferably 0.01 mg or 0.0002 mg/kg, more preferably 0.1 mg or 0.002 mg/kg) and at most 2000 mg or 40 mg/kg (preferably 200 mg or 4 mg/kg, more preferably 20 mg or 0.4 mg/kg), which can be administered at one time or divided into several doses.

Furthermore, when a GLP-1 receptor agonist that has physical properties for excellent sustainability in the blood or a formulation that improves sustainability in the blood is selected, the GLP-1 receptor agonist may be administered at intervals of once or several times per month or one to several times per week. Administration of the GLP-1 receptor agonist at such intervals is also included in the combined use of the present invention, and a pharmaceutical composition containing a GLP-1 receptor agonist and/or compound A that is produced with the intention of this combined use is also included in the present invention.

The amount of compound A contained in the above-mentioned pharmaceutical formulations is not particularly limited, but is suitably selected in a wide range. It is usually suitable to include compound A in an amount of 1 to 70% by weight, preferably 1 to 30% by weight, of the total composition.

The dose varies depending on symptoms, age, body weight, dosage form, and the like, and the usual daily dose for an adult is at least 0.001 mg or 0.00002 mg/kg (preferably 0.01 mg or 0.0002 mg/kg, more preferably 0.1 mg or 0.002 mg/kg) and at most 2000 mg or 40 mg/kg (preferably 200 mg or 4 mg/kg, more preferably 20 mg or 0.4 mg/kg), which can be administered at one time or divided into several doses.

In the present invention, a GLP-1 receptor agonist and compound A are administered at the above-mentioned doses and administration intervals simultaneously or separately at different times. When these compounds are separately administered, the administration route is not particularly limited so long as the administration route is suitable for each compound. Preferred examples of the administration route of compound A include oral or intravenous routes, and preferred examples of the administration route of the GLP-1 receptor agonist include intravenous, intranasal, and subcutaneous routes.

Furthermore, the dose of each drug for one administration can be reduced by the combined use of the present invention, whereby adverse drug reactions attributable to each drug can be reduced.

According to the present invention, a synergistic blood sugar lowering effect against high blood sugar levels in diabetes can be exhibited by using a pharmaceutical composition comprising a GLP-1 receptor agonist and the insulin resistance improving agent 5-[4-(6-methoxy-1-methyl-1H-benzimidazol-2-ylmethoxy)benzyl]thiazolidine-2,4-dione, or a pharmacologically acceptable salt thereof, in combination, and an excellent effect of improving dysfunctions of pancreatic β cells can be exhibited, so that effective prophylactic and therapeutic treatment of diabetes is enabled. Furthermore, an excellent effect of improving insulin resistance can be expected for administration of this pharmaceutical composition. Furthermore, this pharmaceutical composition is effective for diabetic complications caused by high blood sugar levels. Furthermore, rapid improvement of high blood sugar levels as well as a stable blood sugar lowering effect in long-term treatment can be expected by suitably selecting the type, administration method, dose, and the like of each drug depending on the symptom, and a prophylactic and therapeutic agent for the above-mentioned disease that hardly causes adverse drug reactions can be obtained. Moreover, it appears that the dose of each drug for one administration can be reduced by using these drugs in combination, whereby adverse drug reactions attributable to each drug can be reduced.

### Brief Description of the Drawings

Figure 1-A is a graph showing blood sugar levels in full feeding in Test Example 1; Figure 1-B is a graph showing HbA1c levels in Test Example 1; Figure 1-C is a graph showing increases in body weight in Test Example 1; and Figure 1-D is a graph showing the pancreatic insulin content in Test Example 1. In these graphs, indications above bars show results of statistical analyses. According to the results of Tukey's multiple comparison test, "a" was given when a significant difference (P < 0.05) was obtained in comparison with the control group, "b" was given when a significant difference (P < 0.05) was obtained in comparison with group A, and "c" was given when a significant difference (P < 0.05) was obtained in comparison with group B. Furthermore, "A" was given for the result showing a significant interaction between compound A and GLP-1 (7-36) amide by two-way analysis of variance.

### Best Mode for Carrying Out the Invention

The present invention will be explained more specifically with reference to the following examples. However, the scope of the present invention is not limited to these examples.

### Examples

Hydrochloride salt of 5-[4-(6-methoxy-1-methyl-1H-benzimidazol-2-ylmethoxy)benzyl]thiazolidine-2,4-dione (compound A), an insulin resistance improving agent, can be produced according to the methods described in Japanese Patent Laid-Open No. 9-295970, European Patent No. 0745600, U.S. Patent No. 5,886,014, and International Patent Publication WO00/71540.
GLP-1 (7-36) amide can be produced according to the method described in U.S. Patent No. 5,614,492.

Test Example 1: Effect of combined use of GLP-1 (7-36) amide and compound A

### (1) Animals

In this test, commercially available diabetic obese rats (male ZDF rats, 8 weeks old at the start of administration, purchased from Charles River Laboratories Japan, Inc.) were used. ZDF rats used in this test were acclimatized to the feeding time from 9:30 to 16:30 from one week before the start of treatment.

### (2) Experimental methods and results

Rats were divided into four groups: the control group, compound A treatment group (group A), GLP-1 (7-36) amide treatment group (group B), and compound A plus GLP-1 (7-36) amide treatment group (group C).

The control group and group B were allowed free access to a powder feed (FR-2: Funabashi Farms Co., Ltd.) from 9:30 to 16:30. Groups A and C were allowed free access to FR-2 containing compound A at 0.000025% from 9:30 to 16:30 every day. The dose of compound A was calculated as 16.1 µg/kg/day based on the food intake during this period.

Groups B and C were subcutaneously given a GLP-1 (7-36) amide solution prepared with physiological saline to make 100 µg/ml at a dose of 100 µg/kg at 9:30 and 16:30 every day. Similarly, the control group and group A were subcutaneously given physiological saline.

Glycosylated hemoglobin (HbA1c) levels were measured at 42 days after the start of treatment, and blood sugar levels in full feeding were measured at 47 days. Furthermore, animals were dissected at 57 days after the start of treatment to measure organ weight and the content of insulin in the pancreas (pancreatic insulin content). Furthermore, increases in body weight were obtained based on the body weights at the start of treatment and at 57 days. These results are shown in Figure 1.

Since glycosylated hemoglobin is produced when blood glucose and hemoglobin are non-enzymatically bound, and its proportion is increased by prolonged high blood sugar levels, it is widely used as an indicator in long-term treatment (blood sugar control) of diabetic patients (New England Journal of Medicine [N. Eng. J. Med.], Vol. 310, pp. 341-346 [1984]).

Furthermore, insulin in the pancreas was extracted by the hydrochloric acid-ethanol method (Diabetelogia, Vol. 27, pp. 454-459 [1984]), and the insulin concentration in the extract was determined by using a rat insulin RIA kit manufactured by Linco Research, Inc. to obtain the pancreatic insulin content. The pancreatic insulin content is widely used as an indicator showing the condition of pancreatic β cells, insulin secreting cells, and is known to decrease with the worsening of diabetes (Proceedings of the National Academy of the Sciences of the United States of America [Proc. Natl. Acad. Sci. U.S.A.], Vol. 96, pp. 10857-10862 [1999], Diabetes, Vol. 48, pp. 2398-2406 [1999], etc.).

Blood was collected from the caudal vein of the rats, and blood sugar levels were measured with Glucoroder GXT (A & T Corporation). HbA1c levels were measured with DC2000 (Bayer Medical Ltd.).

The results of this test showed a synergistic decrease in HbA1c levels by the combined use of compound A and GLP-1 (7-36) amide (results of two-way analysis of variance: Figure 1-B). A large decrease in blood sugar levels in full feeding was observed in the combined use, as compared with the use of each drug alone (Figure 1-A). Furthermore, weight gain by treatment with compound A was suppressed by using this compound in combination with GLP-1 (7-36) amide (Figure 1-C). A large increase in the pancreatic insulin content was observed by the combined use, as compared with the use of each drug alone (Figure 1-D).

The above results suggest that the combined use of compound A and a GLP-1 agonist is useful as a diabetes treatment.

### Test Example 2: Effect of combined use of various GLP-1 receptor agonists and compound A

By performing tests in the same manner as Test Example 1 using GLP-1 (7-37), Arg³⁴Lys²⁶(N⁶-(γ-L-glutamyl(N^{α}-palmitoyl))) GLP-1 (7-37), exendin 3, exendin 4, and the like as GLP-1 receptor agonists, excellent therapeutic effects against diabetes (synergistic effect on decrease in HbA1c levels, effect of suppressing weight gain by compound A by combined use, marked increase in the pancreatic insulin content, etc.) by the combined use of these GLP-1 receptor agonists and compound A can be confirmed. Each GLP-1 receptor agonist can be produced as described above.

Furthermore, the insulin resistance improving effect of each administration can be determined by measuring fasting blood sugar levels and fasting plasma insulin levels in Test Example 1. Plasma insulin levels can be determined by RIA using Insulin RIA Kit (Linco Research, Inc.).

### Industrial Applicability

The pharmaceutical composition comprising or the therapeutic method using a GLP-1 receptor agonist and 5-[4-(6-methoxy-1-methyl-1H-benzimidazol-2-ylmethoxy)benzyl]thiazolidine-2,4-dione, an insulin resistance improving agent, in combination provided by the present invention is useful in effective prophylactic and/or therapeutic treatment of diabetes.

### [Sequence listing free text]

SEQ ID NO: 2: Amino acid sequence of exendin 4
SEQ ID NO: 3: Amino acid sequence of exendin 3

## Claims

1. A pharmaceutical composition comprising a GLP-1 receptor agonist and 5-[4-(6-methoxy-1-methyl-1H-benzimidazol-2-ylmethoxy)benzyl]thiazolidine-2,4-dione or a pharmacologically acceptable salt thereof in combination.

2. The pharmaceutical composition according to claim 1, wherein the GLP-1 receptor agonist is a GLP-1 related peptide.

3. The pharmaceutical composition according to claim 2, wherein the GLP-1 related peptide is GLP-1 (7-37), GLP-1 (7-36) amide, Arg³⁴Lys²⁶(N⁶-(γ-L-glutamyl(N^{α}-palmitoyl))) GLP-1 (7-37), exendin 3, exendin 4, a derivative or analogue thereof, or a pharmaceutically acceptable salt thereof.

4. The pharmaceutical composition according to claim 2, wherein the GLP-1 related peptide is GLP-1 (7-37), GLP-1 (7-36) amide, Arg³⁴Lys²⁶(N⁶-(γ-L-glutamyl(N^{α}-palmitoyl))) GLP-1 (7-37), exendin 3, exendin 4, or a pharmaceutically acceptable salt thereof.

5. The pharmaceutical composition according to claim 2, wherein the GLP-1 related peptide is GLP-1 (7-36) amide or a pharmaceutically acceptable salt thereof.

6. The pharmaceutical composition according to any one of claims 1 to 5, **characterized in that** the GLP-1 receptor agonist is administered via an administration route selected from intravenous, intranasal, and subcutaneous routes, and 5-[4-(6-methoxy-1-methyl-1H-benzimidazol-2-ylmethoxy)benzyl]thiazolidine-2,4-dione or a pharmacologically acceptable salt thereof is administered via the oral administration route.

7. The pharmaceutical composition according to any one of claims 1 to 6, which is a drug for prophylactic or therapeutic treatment of diabetes.

8. The pharmaceutical composition according to claim 7, which is a glycosylated hemoglobin lowering agent.

9. The pharmaceutical composition according to claim 7, which is a blood sugar lowering agent.

10. The pharmaceutical composition according to claim 7, which is an agent for improving pancreatic β cell function.

11. The pharmaceutical composition according to claim 7, which is an insulin resistance improving agent.

12. Use of a GLP-1 receptor agonist and 5-[4-(6-methoxy-1-methyl-1H-benzimidazol-2-ylmethoxy)benzyl]thiazolidine-2,4-dione or a pharmacologically acceptable salt thereof for production of the pharmaceutical composition according to any one of claims 1 to 11.

13. Use of a GLP-1 receptor agonist or 5-[4-(6-methoxy-1-methyl-1H-benzimidazol-2-ylmethoxy)benzyl]thiazolidine-2,4-dione or a pharmacologically acceptable salt thereof for production of the pharmaceutical composition according to any one of claims 1 to 11.

14. A method of therapeutic or prophylactic treatment of diabetes, **characterized in that** a GLP-1 receptor agonist and 5-[4-(6-methoxy-1-methyl-1H-benzimidazol-2-ylmethoxy)benzyl]thiazolidine-2,4-dione or a pharmacologically acceptable salt thereof are administered in combination to a patient with diabetes or a patient who is at risk of having diabetes.


SEQUENCE LISTING

<110>  SANKYO COMPANY LIMITED

<120>  Treatment of diabetes by combination use of drugs

<130>  FP0616

<150>  JP2005-155174
<151>  2005-05-27


<160>  3

<170>  PatentIn version 3.3

<210>  1
<211>  31
<212>  PRT
<213>  Homo sapiens

<220>
<223>  Inventor: Nakashima, Ryutaro; Ogawa, Junko
       Inventor: Okuno, Akira


<400>  1

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  2
<211>  39
<212>  PRT
<213>  Artificial

<220>
<223>  amino acid sequence of exendin-4

<400>  2

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
        35


<210>  3
<211>  39
<212>  PRT
<213>  Artificial

<220>
<223>  amino acid sequence of exendin-3

<400>  3

His Ser Asp Gly Thr Phe Thr Ser Asp Leu Ser Lys Gln Met Glu Glu
1               5                   10                  15

Glu Ala Val Arg Leu Phe Ile Gly Trp Leu Lys Asn Gly Gly Pro Ser
            20                  25                  30

Ser Gly Ala Pro Pro Pro Ser
        35